# EUROPEAN PATENT APPLICATION

(11) **EP 1 447 653 A1**
(43) Date of publication of application: **18.08.2004**
(21) Application number: 02728193.0
(22) Date of filing: 29.05.2002
(51) Int. Cl.: G01L 1/14, H01G 5/00, A43B 7/00, A61G 5/04, A47C 7/62, A61G 7/05, B60N 2/44, A63H 11/00, A61B 5/11

(54) **PRESSURE−SENSITIVE SENSOR AND MONITOR USING THE PRESSURE−SENSITIVE SENSOR**

(30) Priority: 22.10.2001 JP 2001323138
(71) Applicant: Microjenics, Inc., Tonami-shi, Toyama 939-1365 (JP); National Institute of Advanced Industrial Science and Technology, Tokyo 100-0013 (JP)
(72) Inventor: HAYASHI, Kotaro, Tonami-shi, Toyama 939-1365 (JP); MAKIMURA, Takanobu, Tonami-shi, Toyama 939-1365 (JP); SHIBATA, Takanori, Tsukuba-shi, Ibaraki (JP)
(74) Representative: Luderschmidt, Schüler & Partner GbR
(86) International application number: PCT/JP2002/005227
(87) International publication number: WO 2003/036247

(57) **Abstract**

A pressure-sensitive sensor (a pressure sensor) for measuring the pressure applied to a pressure-receiving part by assuming the pressure as an amount of deformation of the pressure-receiving part and converting the amount of deformation into an electrical signal and a monitor for monitoring the action and the state and change of the emotional motion of a person through the pressure-sensitive sensor, for example, a pet robot that senses the petting action of the owner and performs a predetermined reaction and action are disclosed. The sensor comprises at least two parallel electrode sheets disposed in a spaced relationship from each other and an expandable dielectric member interposed between the adjacent two electrode sheets. When a pressure is exerted on the outermost electrode sheet, the dielectric member deforms and its capacitance varies.

## Description

### TECHNICAL FIELD

The present invention relates to a pressure-sensitive sensor (a pressure sensor) for converting an amount of deformation into an electrical signal and detecting the amount of deformation by treating a pressure applied to a pressure-receiving part as the amount of deformation of the pressure-receiving part, to a monitor for monitoring an action as well as the state and changes of an emotional motion of a person through the pressure-sensitive sensor, and to a pet robot for performing a predetermined reaction and action by sensing the petting action of an owner.

### BACKGROUND TECHNOLOGY

As pressure-sensitive sensors, there are known, among others, those of the type that can detect an amount of the pressure applied through a plurality of electrodes superposed on one other with a spacing interposed therebetween within an elastic member by allowing one or more of the facing electrodes to come into contact with each other and transmitting electricity through the electrode sheets by deforming the elastic member due to the application of pressure, and those of the type for converting a pressure applied to a pressure-receiving part into an electrical signal by using a pressure-sensitive resistance member made from a pressure-sensitive resistance member including, for example, a pressure-sensitive and conductive rubbery material and so on and sense the electrical signal.

As the former is of the type that can sense a contact or non-contact between the facing electrodes, however, it cannot sense a minute amount of pressure that causes no transforming the elastic member. As the latter can be used in a limited field where a pressure greater than a predetermined magnitude is applied, on the other hand, because the pressure-sensitive and conductive rubbery material has minute changes in output by the application of such a minute pressure. Moreover, the pressure-sensitive sensors have the structural problems that there is the tendency of the detecting regions are dispersing in a discontinuous fashion within a narrow scope and that non-sensitive points are dispersed even within a region where the sensing of pressure is needed.

In addition, patent applications have already been filed, for example, for inventions on the technology, in which a pressure-sensitive sensor or switch is embedded inside a chair or a bed, for sensing a state as to whether a person is seated or laid on a chair or a bed, for sensing whether the person is a child or an adult by setting a weight, or for changing a shape of the chair or the bed in accordance with comfortableness. It can be appreciated, however, that conventional inventions have been made with the object to perceive the existence of a person, but they have no object to assume the action of a person as well as a state and a change of an emotional motion of the person.

### DISCLOSURE OF THE INVENTION

The present invention has been completed with the above background taken into account, and it has the object to provide a pressure-sensitive sensor capable of sensing for sure a minute pressure without forming any non-sensing band in a region where the sensing of pressure is needed, to provide a monitor, for example, with the pressure-sensitive sensor loaded therein, capable of monitoring the action and the state of an emotional motion of a person, and to provide, for example, a chair or a bed with the monitor loaded therein, a pet robot, and a wearable interface.

The pressure-sensitive sensor according to the present invention made for the purpose to solve the problems as described above is characterized with a sensing part having electrode sheets, including an electrode sheet working as a pressure-receiving face, disposed in a spaced relationship from each other with an expandable tangible dielectric member interposed therebetween. The term "pressure-receiving face" as used herein is intended to mean a face onto which the pressure is actually applied, whether directly or indirectly. The term "expandable" and related terms as used herein are each intended to mean a property of being deformable as well as reproducible and include a concept of being so-called elastic. The term "tangible" of the tangible dielectric member as used herein is intended to mean a property of sustaining a certain stable shape, excluding an unstable state, such as a liquid or gel-like state.

The pressure-sensitive part is composed of a sandwich structure with the electrode sheets and the tangible dielectric member sandwiched therebetween so as to produce an action equivalent of a condenser working as one of major electronic circuit parts. The pressure-sensitive part may comprise a monolayer structure, or a bilayer or polylayer structure, having a pressure-sensitive part comprising three electrode sheets, including the electrode sheet acting as the pressure-receiving face, superposed on one other in a spaced relationship from each other with the expandable tangible dielectric member interposed therebetween.

The tangible dielectric member may be formed in a spongy state so as to become likely to be deformed by the application of a very minute amount of pressure. The tangible dielectric member may be formed by immersion in a liquid dielectric member so as to heighten a dielectric constant of the resulting tangible dielectric member. Moreover, a flexible sheet may be used for the electrode sheet in order to improve adaptability to the environment in which the pressure-sensitive part is disposed. It is to be noted herein that the flexible sheet may be appropriately chosen from sheets having a property capable of being bent and/or twisted, and whether the flexible sheet is provided with reproducibility can be determined on the basis of the field of use. As specific examples of the flexible sheet, there may be mentioned, for example, a flexible substrate with an electrode material such as a copper foil or the like attached to its front face, a conductive sheet with conductive fibers intertwined like an electromagnetic shield material, or a thin plate of a conductive metal.

The pressure-sensitive sensor according to the present invention may be used for a pet robot having one of the above illustrated pressure-sensitive sensors embedded under an outer covering member as well as having a pressure detection means and a reaction means inside the outer covering member, in which the pressure detection means is designed so as to judge the presence and absence of an electrical deformation generated by the application of pressure to the pressure-sensitive part of the pressure-sensitive sensor and to generate a pressure-sensitive signal upon judgment of the presence of the electrical signal generated by the application of the pressure to the pressure-sensitive part, and the reaction means is designed so as to execute a predetermined motion in response to the pressure-sensitive signal.

It is to be noted herein that the term "under the outer covering member" is intended to mean a shallow portion at which the pressure-sensitive sensor can function as a pressure-sensitive sensor and it does not always physically mean to be immediately under the outer covering member. As the outer covering member, there may be mentioned, for example, a cloth, a sheet member or a plate member, disposed to cover the outermost shell of an article, such as a cover sheet, a protective sheet or a decorative sheet. In the case where a stuffed toy is taken as an example, the outer covering member corresponds to a knit, felt or woolen cloth, for example, disposed as a skin. Further, the electrical deformation is intended to include a deformation with regard to a predetermined constant physical amount (an absolute deformation) or a periodical deformation on the basis of a comparison of a physical amount before a predetermined time with a current physical amount (a relative deformation) .

The pressure-sensitive sensor according to the present invention may be used for a chair and a bed and as a wearable interface.

### Regarding a chair:

The pressure-sensitive sensor can sense the presence of a person on the basis of the manner of seating on the chair. It can also measure a size of the person on the basis of the area on which the pressure is exerted and estimate the weight of the person on the basis of the pressure exerted per area. Such calibrations may be carried out daily to recognize the persons who are seated and monitor a variation of the weights of the persons and other parameters.
(1) Regarding a sheet of a seat for exclusive use with a car:
   In the case where a variation in a periodical low frequency is detected by focusing attention on a variation in pressures exerted from the back side of a person carrying out operations, such as driving the car, a judgment is made such that the person driving the car is most likely to fall into a doze.
   (a) Input: A plane pressure-sensitive sensor is embedded each in a seated face and a back face of the seat to detect the pressure exerted thereon. Information from sensors other than the seat sensors may be combined.
   (b) Information processing: A state of the person sitting on the seat is assumed by measuring the total sum of the pressure exerted onto the sitting seat face or the back seat face or a variation in the pressure per unit face with an elapse of time and comparing the motion of the seated person with a human's motion model by focusing on periodicity or the like.
   (c) The control of other devices is carried out depending on the assumed state of the driver so as to automatically let a machine act onto the driver or assist the driver.

   For example, when the car is driven at a low speed in combination with car speed information, a fluctuation in the motion of a person can be detected to judge as to whether the person becomes nervous. If it is judged that the person is irritating, then the car can act on the person so as to cool down in association with a massaging function and/or a configuration-changing function disposed in the seat and/or other information media and so on.
(2) Regarding a seat for an airplane:
   Monitoring is carried out as to whether a passenger's motion changes in an appropriate fashion. If the passenger do not move for a predetermined period of time, a signal can be given the passenger to move, for example, in order to prevent an occurrence of problems causing an economy syndrome or other problems that might be caused to occur due to staying in an identical posture for a long time.

### Regarding an office chair:

(1) An office chair may be provided with a built-in plane pressure-sensitive sensor in the sitting face and the back face of the chair. This built-in plane pressure-sensitive sensor can monitor the posture of the person in a state of sitting on the chair or a variation in the posture thereof. Monitoring the posture and the variation of the posture of the person can assist in managing the working state and checking the working time of a person sitting and working before a computer. Further, monitoring the motion of the body of a working person can assist in the management of an extent of fatigue or a health control of the person. In addition, the chair can provide an active service by positively moving the chair, massaging or other services, in the case where a person is working while sitting on the chair continuously during a long period of time.
(2) While a person is absent from the chair in office for a long time, a telephone call to a fixed telephone can automatically be transferred from the fixed telephone to another telephone including a portable telephone or the like if the fixed telephone would give no answer for a predetermined period of time. Further, as the receiver is back to his office and seated on the chair, the transfer is terminated within short and the telephone line is switched and connected to the fixed telephone.

### Regarding a bed:

A pressure-sensitive sensor disposed in a bed can recognize a person sleeping on the bed on the basis of a posture of the sleeping person. The pressure-sensitive sensor measures a size of the person on the basis of an area on which the pressure of the person is exerted and presume the weight of the sleeping person on the basis of a pressure per unit area exerted from the person sleeping on the bed. This data is measured on a daily basis to recognize the person and monitor a variation in the weight of the person and other parameters.
(1) A plane pressure-sensitive sensor is embedded in a mat of a bed to monitor the state in which person is sleeping. Further, monitoring as to whether the person sleeping on the bed on a periodical basis can prevent the death of an old person staying in an old-age home or a baby sleeping with the face lied face downward.
(2) A plane pressure-sensitive sensor is embedded in a coverlet in order to recognize whether the coverlet is placed on the sleeping person. The pressure-sensitive sensor can sense a contact of the coverlet with the body of the sleeping person by monitoring a variation in the pressure exerted on the coverlet. For example, if the coverlet would fall from the bed side, this state can be detected due to the fact that no pressure to be exerted on the coverlet varies any longer.

### Regarding a key board:

A plurality of plane pressure-sensitive sensors are used for an input device for entering letters including, for example, alphabets and kana letters. Letters can be chosen at a high speed by changing the speed at which letters are changed due to changes in pressure. A pressure-sensitive sensor can also be used as a switch of a portable telephone for making an input of letters variable.

### Regarding an electronic wheelchair:

The pressure-sensitive sensor for use with an electronic wheelchair can control the direction and the speed of moving the wheelchair by moving the body of the person. A plane pressure-sensitive sensor is embedded in the sitting face of the seat of the electronic wheelchair. An instruction on the direction of advancement of the wheelchair can be given to the wheelchair by moving the gravity of the weight of the driver in all directions, i.e., forwards, rearwards, leftwards and rightwards. The pressure-sensitive sensor can be used as a replacement for an existing joystick.

### Regarding shoes:

Checking the way of walking (in order to prevent the hallux valgus) : A plane pressure-sensitive sensor is embedded in inner soles of shoes to monitor a portion of the sole of the foot on which the body weight is exerted from the person wearing the shoes. This allows checking a manner of standing or walking. While a person is standing while working for a long time, a balance of the body weight exerted on the left and right feet is monitored to give the person working in a standing posture an instruction on the necessity of resting or other information. Further, the pressure-sensitive sensors disposed in the shoes can monitor the time of walking and give the walker an advice for resting or information on abnormality in the manner of walking as the weight starts inclining towards the outside of the foot as time elapses. These functions can prevent an occurrence of the hallux valgus and check a manner of walking for an old person, thereby preventing a crick in the back or the like.

### Brief Description of the Accompanying Drawings

Figs. 1(a) and 1(b) are schematic illustrations each showing an example of a state of a variation due to stress of a pressure application part in the pressure-sensitive sensor according to the present invention.
Fig. 2 is a schematic illustration showing an example of a state of connection of the pressure-sensitive sensor according to the present invention to a pressure detection means.
Fig. 3 is a schematic illustration showing an example of a pressure-sensitive sensor unit including the pressure-sensitive sensor according to the present invention.
Fig. 4 is a schematic illustration showing an example of disposition of the pressure-sensitive sensor according to the present invention.
Fig. 5 is a circuit drawing showing an example of a pressure detection means adopted for a pet robot using the pressure-sensitive sensor according to the present invention.
Figs. 6(a), (b), (c), (d), (e) and (f) are each a plan view showing a cut example of the pressure-sensitive sensor according to the present invention.

### Best Modes for Carrying out the Present Invention

A description will now be given regarding the modes of carrying out the pressure-sensitive sensor according to the present invention as well as the modes of carrying out a pet robot using the pressure-sensitive sensor, with reference to the accompanying drawings.

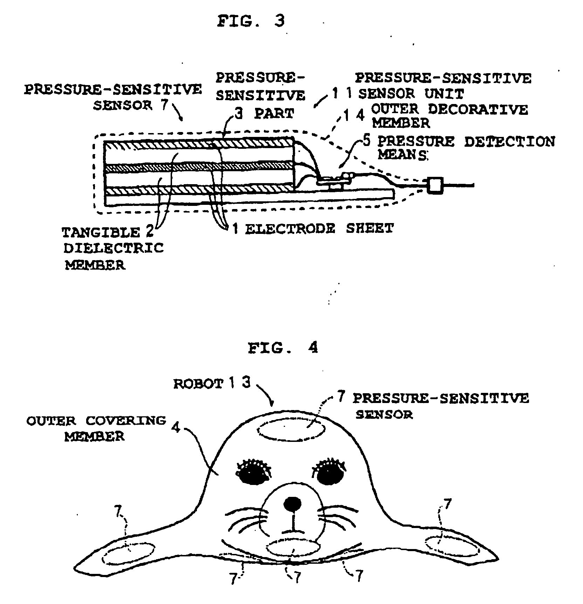

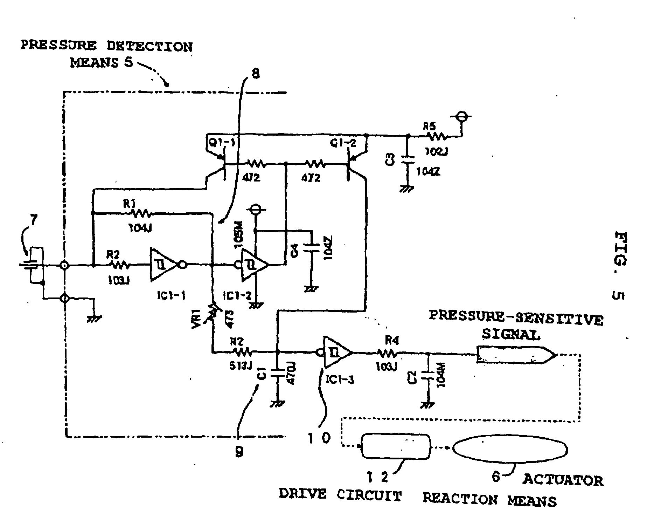

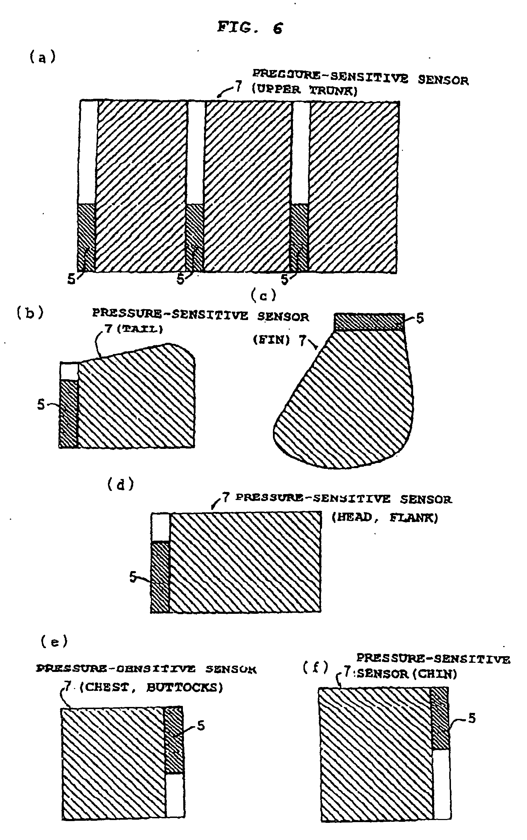

The embodiment as shown in Fig. 4 is directed to a stuffed toy 13 in the shape of a seal (hereinafter referred to as a "robot"). The robot 13 is designed in such a manner that a pressure-sensitive sensor 7 (as shown in Fig. 6) is embedded each at portions under an outer covering member 4, corresponding to the trunk, tail, fin, head, flank, chest, buttocks and chin thereof, so as to sense an electrical signal generated by the pressure applied to the respective portions of the body of the toy 13. As shown in Fig. 5, the robot is provided with a pressure detection means 5 and a reaction means 6 inside the outer covering member, in which the pressure detection means is designed so as to calibrate a physical amount indicating an electrical deformation caused by a variation in pressure applied by a pet stroke, hits or the like and sensed by the pressure-sensitive sensors 7 and to generate a pressure-sensitive signal in accordance with a magnitude of the physical amount, and the reaction means is designed so as to execute a predetermined motion in response to the pressure-sensitive signal.

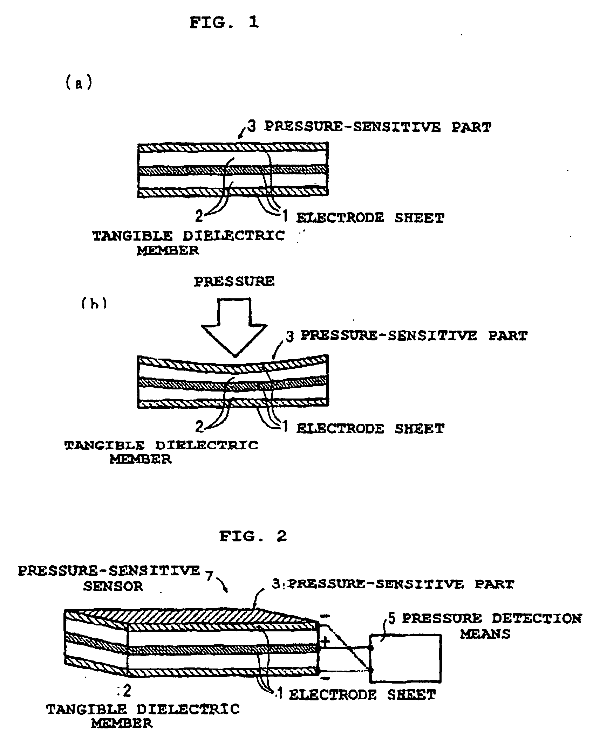

As shown in Figs. 1 to 3, the pressure-sensitive sensor 7 comprises electrode sheets 1 and 1 disposed in a spaced relationship, including an electrode sheet 1 acting as a pressure-receiving face interposed between therebetween, and a tangible dielectric member 2 made out of a urethane sponge having an expandable property. The pressure-sensitive sensor also comprises a pressure-sensitive part 3 composed of the electrode sheets 1, 1, disposed in a spaced relationship with the electrode sheet 1 acting as the pressure-receiving face interposed therebetween and the expandable tangible dielectric member 2, and each of the electrode sheets 1 is connected to a circuit of a pressure detection means 5. The electrode sheet 1 is formed with a thin plate from a raw material including, such as copper or the like, and it in itself can play a role equivalent of a counter-electrode of a condenser. As the electrode sheet 1, there may be used, as needed, a flexible sheet made from a woven cloth or an unwoven cloth, each made out of knitted wires or conductive fibers so as to enable the pressure-sensitive sensor 7 to be embedded under the outer covering member 4 of the robot in a curved state.

In this embodiment, the tangible dielectric member 2 having an expandable property is interposed among each of three electrode sheets 1, 1 and 1, superposed in a spaced relationship from one another. This structure can provide an electrical property equivalent of that of two wide condensers connected in series to each other through the central electrode sheet 1. It is to be provided herein, however, that an electrically parallel connection can be achieved in the case where an output is taken from the second electrode 1 inside of the three electrodes 1, 1, 1, as shown in Figs. 2 and 5, and the outside two electrode sheets 1 and 1 are connected to a GND of the pressure detection means 5. In this embodiment, the urethane sponge may preferably comprise, for example, a urethane sponge having density of approximately 20 kg/cm³ and hardness of approximately 19. 6 N, while a feel upon application of pressure or reproducibility after deformation is taken into account. These parameters can be gained by impregnating the tangible dielectric member 2 in a liquid dielectric member, as needed. In such a case, however, it is needed to sufficiently seal the pressure-sensitive sensor 7 with a sheathing material 14 composed of an insulating material having a low dielectric constant.

As shown in Fig. 5, the pressure detection means 5 as used in this embodiment comprises an oscillation circuit 8 designed to set an oscillating cycle depending upon the capacitance of the pressure-sensitive sensor 7, a charging-and-discharging circuit 9 designed so as to repeat the charging and the discharging at every oscillating cycle of the oscillation circuit, and a pressure detection circuit 10 designed so as to detect a magnitude of the pressure applied against the pressure-sensitive sensor 7 in comparison of the potential accumulated in a condenser C1 of the charging-and-discharging circuit 9 with a predetermined threshold value.

The charging-and-discharging circuit 9 is disposed in such a manner that, in the case where the discharge is conducted at a cycle of the oscillation circuit 8 at the time when no stress is applied to the pressure-sensitive part 3 of the pressure-sensitive sensor 7 (this instance being hereinafter referred to as "stationary time"), a constant of CR is fixed to such an extent that the lowest potential of the condenser of the charging-and-discharging circuit 9 does not become lower than a threshold value of the pressure detection circuit 10. On the other hand, in the case where a stress is caused to occur due to the pressure exerted on the pressure-sensitive sensor 7, the oscillation circuit 8 causes a change (decrease) in the electrostatic capacitance as the condenser of the pressure-sensitive sensor 7 in accompany with the occurrence of the stress, and a transmitting frequency of the oscillation circuit 8 is affected. In other words, a sample circuit as shown in Fig. 9 elongates a cycle in accordance with a magnitude of the pressure applied upon the application of the stress to the pressure-sensitive sensor 7, as compared with the cycle at the stationary time.

As the oscillating cycle of the oscillation circuit 8 elongates, the discharging period of time becomes extended by the elongation of the discharging time of the charging-and-discharging circuit 9 and the lowest potential of the condenser becomes lower than that at the stationary time as well as lower than the threshold value of the pressure detection circuit 10. As a result, a reverse output stage of the pressure detection circuit 10 outputs a HI level only during the period of time when the threshold value becomes lower, out of the period of time during which the oscillating cycle becomes elongated. In this case, a change of a duty ratio in the oscillating cycle is converted into a predetermined potential via an integrating circuit 15 and outputted as an analog non-stage pressure-sensitive signal in accordance with the magnitude of the pressure applied. It is to be provided, however, that, as the lowest potential of the condenser of the charging-and-discharging circuit 9 does not become lower than the threshold value of the pressure detection circuit 10 in a stationary state, a LOW level is outputted as a pressure-sensitive signal from the reverse output stage of the pressure detection circuit.

The sample circuit is disposed in such a manner that a stress produced in the pressure-sensitive sensor 7 is converted into an electrophysical amount, that is a variation in the capacitance due to the structure of the condenser of the pressure-sensitive part 3, and finally the absolute amount as the lowest potential of the condenser in the charging-and-discharging circuit 9 is compared with a threshold value that is an absolute amount defined by the pressure detection circuit 10. This is referred to as an "absolute amount comparison type". In some cases, however, the pressure detection means 5 may adopt a pressure detection circuit for comparing a variant portion of the electrical deformation, such as a cycle, potential, pulse width, phase and so on, corresponding to a stress to be produced in the pressure-sensitive sensor at a periodical interval, under cases taken into account where the shape of the pressure-sensitive part 3 of the pressure-sensitive sensor can not be recovered at the stationary time due to stresses produced at very frequent occasions and the capacitance cannot be returned to that at the stationary time or where the pressure-sensitive sensor has already been forced to be deformed at the time of embedding. This is referred to as a relative amount comparison type".

As shown in Fig. 5, the sample circuit can judge versatile states including, such as caressing, massaging, patting and so on, and the presence or absence of stress by conducting a stepwise judgment of an analog pressure-sensitive signal outputted from the pressure detection circuit 10 in the potential thereof. Specific techniques for adopting for the pressure detection circuit may be appropriately selected from the existing techniques including, for example, technique for detecting the potential by stages in the manner as described above, technique for detecting the change in the frequency caused by the addition of the stress in binary or multiple stages, and technique for detecting the change in a phase from the stationary state caused by the addition of the stress in binary or multiple stages.

In the embodiment of the sample circuit as described above, as measures for noises, an output is taken from the second electrode sheet 1 interposed between the two outside electrode sheets 1 and 1, as shown in Figs. 2 and 5, and the two outside electrode sheets 1 and 1 are connected to a GND of the pressure detection means 5. It is provided, however, that a variety of pressure detection means 5 may be disposed integrally with each of the pressure-sensitive sensors 7, as shown in Figs. 3 and 6, in order to enable a sure detection of any minute variation in capacitance in accordance with the structure and use of the pressure-sensitive sensor 7 without interference from noises, and it may be embedded as a pressure-sensitive sensor unit 11 connecting the pressure detection means 5 to the pressure-sensitive sensors 7 through the shortest possible line.

The pressure-sensitive sensor unit 11 may be provided at least with a pressure detection circuit for detecting the state of the application of the pressure in the same manner as the pressure detection circuit 10 by the various techniques as described above, in the manner as shown in the sample circuit. In the case where the judgment of a multi-stage level is needed, it may be provided with a level judgment circuit (although not shown), such as an A/D converter, for allocating the pressure detected by the pressure detection circuit in stages to convert the output of the level judgment circuit as a pressure-sensitive signal. Further, in the case where it is considered appropriate to use, for the reaction means 6, particularly an LED or the like, which is relatively small in scale and simplified, a drive circuit 12 of the reaction means 6 including, for example, a current driver or a pulse generating circuit, may be disposed. As the output of the level judgment circuit which is allocated in stages, there may be used, for example, an analog amount such as voltage or the like or a digital value consisting of serial data or parallel data.

For instance, the pressure-sensitive signal from the pressure detection circuit 10 in this embodiment may work as a boosting signal for the drive circuit 12 for a corresponding display device or a corresponding actuator as the reaction means 6 to let the nose, the eyes, the front legs, or the rear legs of the robot 13, for example, take an action in synchronism with the predetermined reaction. It is provided, however, that the pressure-sensitive sensor 7 can be used by cutting the pressure-sensitive part into an appropriate shape so as to fit in the embedding location, as shown in Fig. 6, and it can be used in compliance with design needs by changing the design in a relatively simple manner in accordance with the cut area even in the case where the pressure detection circuit of the absolute amount comparison type is adopted as well as in the case where the pressure detection circuit of the relative amount comparison type is adopted.

### Industrial Utilizability

As described above, the use of the pressure-sensitive sensor according to the present invention can achieve the detection of the status of the application of pressure at low costs and for sure without using an expensive deformation gauge or pressure sensor and in the absence of a non-sensitive band over the full area of the pressure-sensitive part. Further, the pressure-sensitive parts of versatile sizes can be manufactured in accordance with specifications. Moreover, the structure of the pressure-sensitive part, which is composed of the three electrode sheets, including the electrode sheet acting as the pressure-receiving face, arranged in a spaced relationship from one another with the expandable tangible dielectric member interposed between the two outside electrode sheets, enables measures of noises by using the two sheets positioned outside as the GND. In addition, the molding of the tangible dielectric member in a spongy form can relatively easily change a design of a pressure band region to be detected and the kind of the application of pressure (i.e., the spontaneous application of pressure, the continuous application of pressure, etc.) by adjusting a foaming density of the tangible dielectric member. Moreover, the pressure-sensitive part can be loaded even in a curved state by using a flexible sheet for the electrode sheet.

Further, the pet robot using the pressure-sensitive sensor according to the present invention can represent the reaction and the action in a definite and sure way and at a quick timing by surely detecting the state of the application of pressure to the surface part by the pressure-sensitive sensors, thereby allowing an attachment to the robot as compared with an actual living thing. Moreover, the adoption of the periodically relative deformation by using the state of the application of pressure as the electrical deformation provides for convenience with respect to the disposition and the control of the pressure-sensitive sensor, thereby allowing the detection of the application of pressure over every corner of the surface part of the pet robot. This fine detection can realize an extremely fine reaction and action of the pet robot in communication with a person as if the pet robot be an actually living being.

## Claims

1. A pressure sensor comprising at least two electrode sheets arranged in a parallel and spaced relationship from each other and an expandable dielectric member interposed between the two adjacent electrode sheets, wherein capacitance is allowed to vary due to deformation of the dielectric member upon application of pressure to the outermost electrode sheet.

2. The pressure sensor as claimed in claim 1, wherein the dielectric member is molded in a spongy form.

3. The pressure sensor as claimed in claim 2, wherein a liquid dielectric member is impregnated in the spongy dielectric member.

4. The pressure sensor as claimed in any one of claims 1 to 3, wherein each of said electrode sheets is flexible.

5. The pressure sensor as claimed in any one of claims 1 to 4, further comprising a detection means for generating an output signal, connected to each of said electrode sheets, by detecting a variation in capacitance of the dielectric member.

6. A pet robot comprising a main body having at least one action part for conducting a predetermined action in response to an instruction signal, a cover covering at least a portion of the main body, a pressure-sensitive sensor as claimed in any one of claims 1 to 5, embedded in the main body and covered with the cover, wherein the action part is electrically connected to a detection means and is disposed to receive an output signal from the detection means as an instruction signal.

7. The pet robot as claimed in claim 6, wherein said variation in capacitance is a variation between a current time point and a previous time point in a predetermined time before the current time point.

8. A pressure-receiving structural member comprising a main body, a cover covering at least a portion of the main body, and the pressure sensor as described in at least claim 5, embedded in the main body and covered with the cover.

9. The pressure-receiving structural member as claimed in claim 8, wherein said main body is one member selected from the group consisting of a stair chair, a walking chair, an automatic chair, a bed, a coverlet, shoes and a keyboard.
